# EUROPEAN PATENT APPLICATION

(11) **EP 2 364 645 A1**
(43) Date of publication of application: **14.09.2011**
(21) Application number: 11154616.4
(22) Date of filing: 16.02.2011
(51) Int. Cl.: A61B 5/117, G06K 9/00

(54) **Finger vein authentication unit**

(30) Priority: 12.03.2010 JP 2010055293
(71) Applicant: Hitachi Ltd., Tokyo 100-8280 (JP)
(72) Inventor: Kitane, Keiji, Tokyo 100-8220 (JP)
(74) Representative: MERH-IP Matias Erny Reichl Hoffmann

(57) **Abstract**

The present invention provides a finger vein authentication unit 101 which solves problems such that a turn of a finger 4 around the finger as an axis cannot be detected from an image of the finger 4, a finger cannot be placed normally because the nail is caught in a dent portion, and an image of the contour of a finger 4 cannot be obtained by an image capturing method using an image pickup window W which is narrower than a finger 4 and is completely closed with a finger so that a turn of a finger cannot be detected from the counter. A touch sensor 5 having an X-Y matrix capable of obtaining the position and shape of a fingertip is mounted in a fingertip placement part, and the fingertip placement part is formed in a flat shape so that a finger is easily put. From the data of the position and shape of the fingertip read by the sensor 5, a turn of the finger around the optical axis of the camera 6 as a center, a turn of the finger around the finger as an axis, and a positional deviation of the finger are detected. On the basis of the data, the vein is extracted accurately and used for a guidance to the user so that the user can put his/her finger in the correct position.

## Description

### BACKGROUND OF THE INVENTION

### 1. Field of the Invention

The present invention relates to a finger vein authentication unit.

### 2. Description of the Related Art

In the biometric authentication technique, since vein authentication uses a vein pattern in a living body, it is more difficult to perform falsification and manipulation as compared with finger print authentication. A safer biometric authentication unit can be realized with the vein authentication. A finger vein authentication unit using a finger vein pattern can be miniaturized more than other vein authentication units such as a palm vein authentication unit and is easily assembled in equipment, so that it is expected to be applied to various fields.

The finger vein authentication unit captures an image of the vein by capturing an image of a finger by an infrared camera having near-infrared light sensitivity by emitting near infrared light to the finger by using the fact that the vein of a finger absorbs near infrared light and the portion other than the vein transmits near infrared light, and uses the vein pattern for authentication.

Japanese Unexamined Patent Application Publication No. 2002-83298 discloses a person authentication unit using finger vein authentication, which detects finger contour from a captured image and corrects a turn of a finger image captured.

Japanese Unexamined Patent Application Publication No. 2003-30632 and Japanese Unexamined Patent Application Publication No. 2006-155575 disclose a configuration of determining a finger placement position by guiding means which guides a finger to a placement position and a button switch provided at a fingertip. Japanese Unexamined Patent Application Publication No. 2007-325793 discloses a configuration of recognizing an outside shape range of a finger by a position sensor having a touch panel and turning on a corresponding near-infrared-light source. The position sensor can detect a turn or tilt of the finger. However, concrete description is not given.

To accurately perform authentication using a vein pattern, it is important to make imaging parameters at the time of registration and those at the time of authentication the same and match a registered vein pattern and a vein pattern to be authenticated as vein patterns of the same part of a finger. In the case where the imaging parameters at the time of registration and those at the time of authentication are different, for example, in the case where a finger placement position in an authentication unit at the time of registration and that at the time of authentication are different from each other, an image of the vein captured by an infrared camera at the time of registration and that at the time of authentication become different from each other, so that vein pat terns extracted look different.

Due to this, false rejection may occur such that although the user himself/herself enters an authentication input, the unit determines that another person enters the input because of the vein patterns which look different. There is also the possibility of occurrence of false acceptance such that although another person enters an authentication input, due to a positional deviation of a finger, a match in the vein patterns is determined by chance.

When the positional deviation between the finger at the time of registration and that at the time of authentication lies in a range of a certain degree, like in the Japanese Unexamined Patent Application Publication No. 2002-83298, there is provided a method of extracting the contour of the finger from a captured image, performing a positional correction on a turn or floating of the finger, or the like on the basis of the contour, and performing authentication. In the case where the finger placement position is largely different from the normal position, it is difficult to make a correction using the contour data.

Further, in a small-sized open-type finger vein authentication unit preventing the influence of outside light by forming the image pickup window narrower than a finger and completely closing the image pickup window with a finger, the contour of a finger is not included in a captured image. Consequently, a positional deviation and a turn of the finger placement position cannot be corrected using the finger contour data.

To avoid the problem, like in the Japanese Unexamined Patent Application Publication No. 2003-30632 and Japanese Unexamined Patent Application Publication No. 2006-155575, there is proposed a finger vein authentication unit which makes the user put his/her finger on the same finger placement position by providing the finger placement part with a switch and a sensor. However, although a turn of a finger in the same plane using the optical axis of the infrared camera as an axis can be prevented, a turn of a finger around the axis of the finger as a center cannot be prevented.

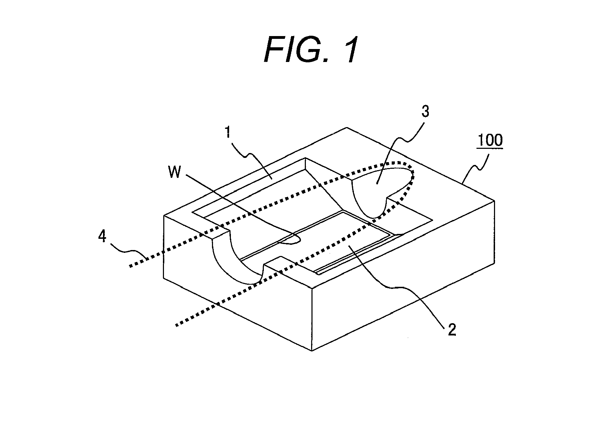

There is also proposed a finger vein authentication unit having a dent in a finger guide part shown in FIG. 1 so that a finger is not deviated from a finger placement part in order to prevent a positional deviation of a finger. Shown in FIG. 1 are a finger vein authentication unit 100, a near-infrared-light emitting unit 1, an image pickup window W provided with a near-infrared-light transmission filter 2, a fingertip guiding part 3, and a finger 4 of the user to be authenticated.

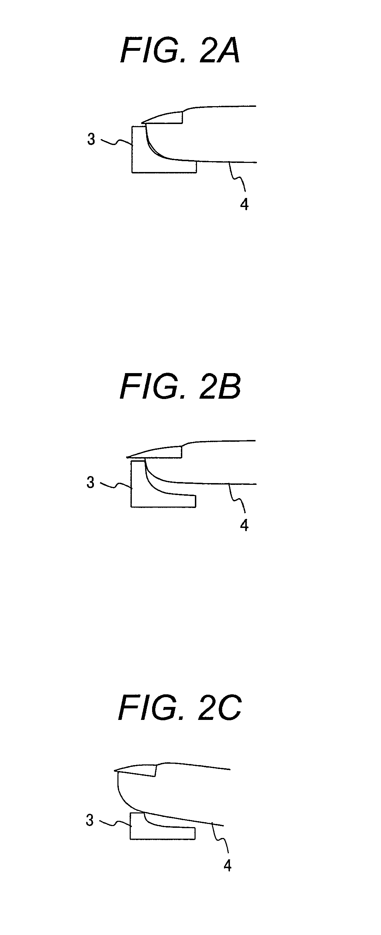

In the finger vein authentication unit 100, when the depth of the fingertip guiding part 3 is increased to prevent a deviation of a finger, a finger having normal thickness can be placed normally as shown in FIG. 2A. However, when a finger has a long nail or an attached nail as shown in FIG. 2B, the nail is caught by the periphery of the fingertip guiding part 3. When the finger is thin, the finger floats and cannot be stably put. When the fingertip guiding part 3 is made shallow so that the nail is not caught, the finger may be put on the periphery as shown in FIG. 2C and a positional deviation increases.

### SUMMARY OF THE INVENTION

The present invention provides a finger vein authentication unit for performing biometric authentication by capturing a finger vein pattern from an image pickup window by an infrared camera using near-infrared light, wherein a touch sensor having a plurality of detection regions which are continuous in XY directions is mounted in a fingertip placement part provided on the outside of the image pickup window, a finger displacement amount from a correct finger placement position in the finger vein authentication unit is detected from fingertip shape data detected by the touch sensor and finger contour data detected by the infrared camera, a finger vein pattern obtained is corrected, and the same finger vein pattern as that at the time of registration to the finger vein authentication unit is automatically obtained.

Further, the finger displacement amount corresponds to a deviation caused by turn of a finger in the same plane around an optical axis of the infrared camera as a center, or a positional deviation in a direction of turn around the axis of the finger as a center or the positional deviation in the axis directions of the finger.

Preferably, the finger placement part has an almost flat shape.

Preferably, when the finger displacement amount exceeds a predetermined threshold value, guidance information is output and notified to the user so that the user puts his/her finger in a correct placement position.

The present invention also provides a finger vein authentication unit for performing biometric authentication by capturing a finger vein pattern from an image pickup window which is closed with a finger by an infrared camera using near-infrared light, wherein a first touch sensor having a plurality of detection regions which are continuous in XY directions is mounted in a fingertip placement part provided on the outside of the image pickup window, a second touch sensor having a plurality of detection regions which are continuous in XY directions is mounted in a finger base placement part provided on the outside of the image pickup window, a finger displacement amount from a correct finger placement position is detected from fingertip shape data and finger base shape data detected by the first and second touch sensors, correction is made, and the same finger vein pattern as that at the time of registration to the finger vein authentication unit is automatically obtained.

Further, the touch sensor is provided with a pressure sensor for detecting a pressing force to the touch sensor.

The touch sensor may be a resistive touch sensor.

In the present invention, a touch sensor having a plurality of detection regions which are continuous in XY directions is mounted in a fingertip placement part provided on the outside of an image pickup window, a finger displacement amount from a correct finger placement position is detected from fingertip shape data detected by the touch sensor and finger contour data detected by an infrared camera, a finger vein pattern is corrected, and the same finger vein pattern as that at the time of registration to the finger vein authentication unit is automatically obtained. Consequently, the user-friendly finger vein authentication unit in which the fingertip placement part is formed in an almost flat shape to make a finger easily put and, simultaneously, authentication precision is high can be realized.

### BRIEF DESCRIPTION OF THE DRAWINGS

FIG. 1 is a perspective view of a conventional general open-type finger vein authentication unit.
FIG. 2A is a schematic diagram of a conventional fingertip guide unit.
FIG. 2B is a schematic diagram of a conventional fingertip guide unit.
FIG. 2C is a schematic diagram of a conventional fingertip guide unit.
FIG. 3 is a perspective view of a first embodiment in which a touch sensor is mounted on a fingertip place
FIG. 4 is a schematic diagram of data obtained by the touch sensor and an infrared camera of the first embodiment.
FIG. 5 is a schematic diagram showing a matching template generating method of the first embodiment.
FIG. 6A is a schematic diagram of finger data by the touch sensor when a finger is not placed normally.
FIG. 6B is a schematic diagram of finger data by a touch sensor when a finger is not placed normally.
FIG. 7A is a schematic diagram of a method of detecting finger turn around the axis of a finger as a center.
FIG. 7B is a schematic diagram of a method of detecting finger turn around the axis of a finger as a center.
FIG. 8 is a schematic diagram of vein data obtained by a finger vein authentication unit of a second embodiment.
FIG. 9 is a schematic diagram of the case where a touch sensor is provided with a pressure sensor in a third embodiment.
FIG. 10 is a schematic diagram showing a contact-type touch sensor in a fourth embodiment.

### DETAILED DESCRIPTION OF THE PREFERRED EMBODIMENTS

Embodiments of the present invention will be described below with reference to the drawings.

### (First Embodiment)

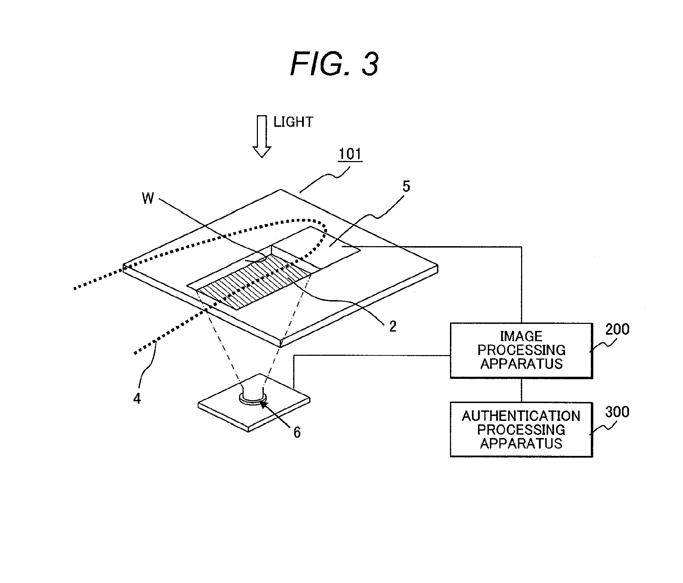

FIG. 3 is a perspective view of a finger vein authentication unit 101 using the present invention. A fingertip placement part has a flat face and, in some cases, may have a dent which is shallow enough to prevent collision of a nail. On the fingertip placement part, a touch sensor 5 which is an electrostatic sensor having a plurality of detection regions divided in an X-Y matrix is mounted. An image of finger vein is captured by an infrared camera 6 provided below via a near-infrared-light transmission filter 2. It is assumed that the near infrared light is emitted from the top face or side face of the finger toward the finger. On the basis of data of the touch sensor 5 and the infrared camera 6, a predetermined matching template is generated by an image processing apparatus 200, and authentication process is performed by an authentication processing apparatus 300. The image processing apparatus 200 and the authentication processing apparatus 300 can be provided on a processor mounted on the finger vein authentication unit 101 or a device such as a PC connected to the processor.

The touch sensor 5 provided for the fingertip placement part can accurately detect a finger contact part and a finger non-contact part and, when a finger is put, obtains data of the position and shape of the fingertip.

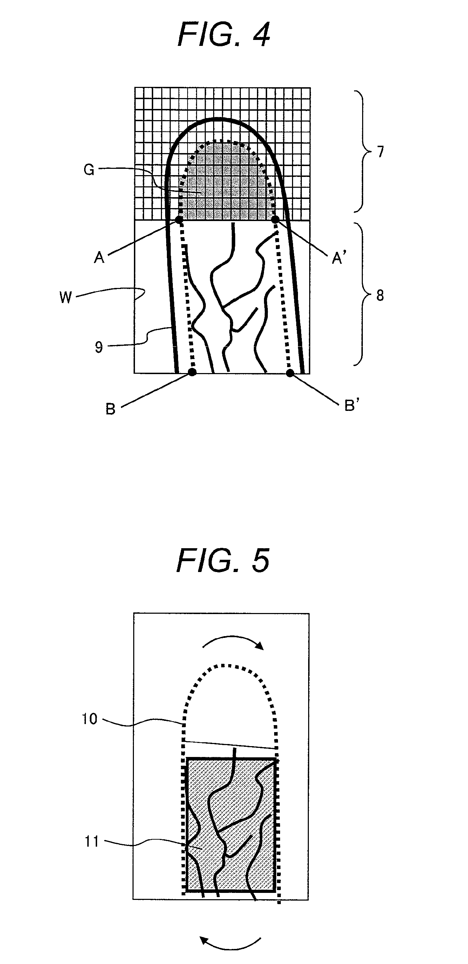

FIG. 4 illustrates data of the finger obtained by the finger vein authentication unit 101 of the first embodiment. When the finger is placed on the finger vein authentication unit 101, as shown in FIG. 4, a gray part G a finger 4 touches is a finger detection part by the touch sensor 5. Since a section of the finger 4 is round, the touch sensor 5 touches the finger in a range narrower than the outline of an image captured by the infrared camera 6, and the finger detected by the touch sensor 5 has a shape smaller than that seen in the image. Reference numeral 7 denotes a touch sensor detection area, and reference numeral 8 denotes an infrared camera detection area.

The infrared camera 6 obtains a vein pattern P of the finger 4 and a contour 9 of the finger as image data. The data of the two kinds is joined and detection portion end points of the finger 4 detected by the touch sensor 5 are set as A and A'. Lines A-B and A'-B' are drawn in parallel to the contour 9 of the finger obtained in the image from the points A and A', and image data on the inside of the lines and data detected by the touch sensor 5 is synthesized to obtain contact contour data 10. As shown in FIG. 5, a template for matching is generated by cutting the vein data in a corresponding position from an image of the entire finger captured by the infrared camera 6 on the basis of the contact contour data 10.

The process will be described concretely. A specific area in the image of the entire finger 4 captured is set as a template generation area 11. At the time of generating a template by cutting the image in the template generation area 11, in the case where the finger 4 is put obliquely, how oblique the finger 4 is put is detected from the contact contour data 10, and a finger vein image is rotated on the image processing apparatus 200 so that the finger becomes almost perpendicular on the basis of the data. Next, by cutting the image in the temperate generation area 11, even when the finger 4 is put obliquely with respect to the finger placement position in the finger vein authentication unit 101, vein data in the same position as that when the finger 4 is put in the normal position can be obtained.

In the case where the finger placement position is deviated from the predetermined position in the axis direction of the finger, the fingertip portion is included in the finger contact contour data 10. Consequently, how much the finger placement position is deviated from the contact contour data 10 in the axis direction is detected, and the finger vein image is moved on the image processing apparatus 200 on the basis of the data. Subsequently, the image in the template generation area 11 is cut out, thereby enabling the vein data in the same position as that at the time of registration to be obtained even when the finger placement position is deviated in the finger axis direction. By correcting turn of the finger and the position in the finger axis direction in the finger contact contour data 10 as described above, even when the finger placement position is deviated slightly, the template for matching can be accurately generated.

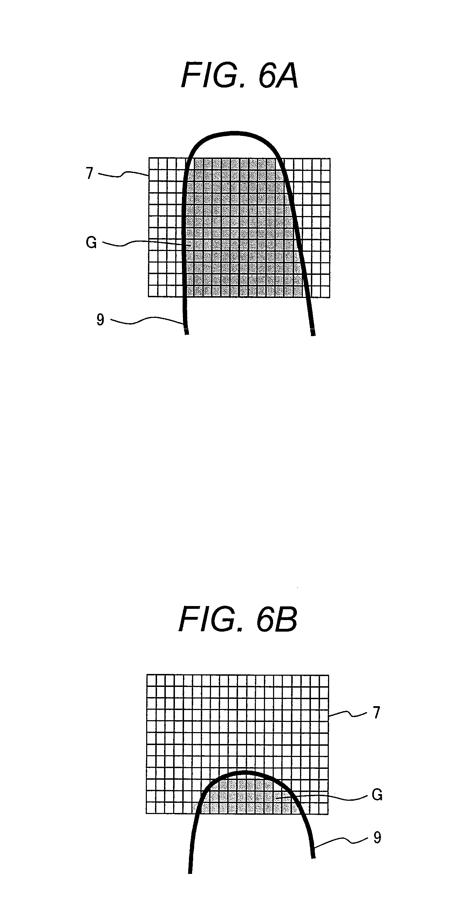

A check is made on the data obtained by the touch sensor 5 in the beginning of registration and authentication. In the case where the fingertip is out of the touch sensor detection area 7 as shown in FIG. 6A and in the case where only a small part of the fingertip is put in the area as shown in FIG. 6B, it is determined that the finger is not put correctly. In this case, the deviation exceeds the above-described automatic correctable range. A guidance is output by sound, characters, a signal, or the like to put the finger in the correct position so that the user can put his/her finger in the correct finger placement position, and data is obtained again.

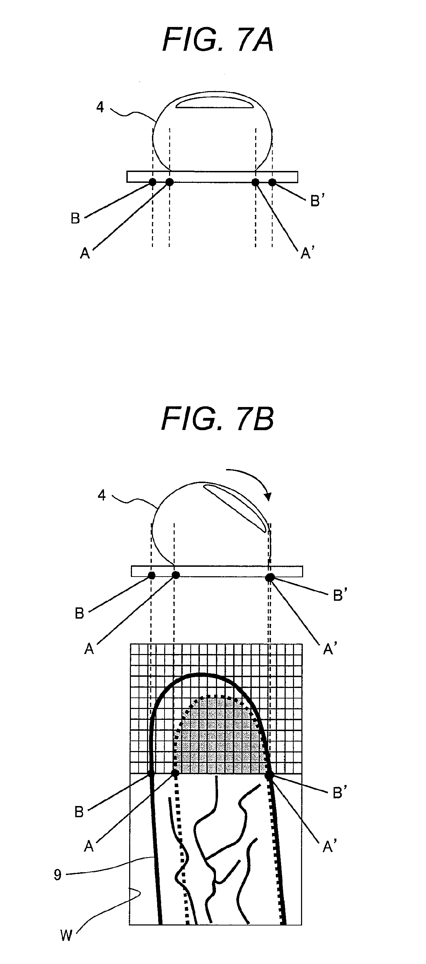

Next, detection of turn of the finger will be described. The contour points of the finger by the touch sensor 5 are set as A and A' as shown in FIG. 7A in the border line between the touch sensor 5 and an image pickup window and the contour points of the finger in the image data are set as B and B'. When the finger is put horizontally, the distance between A and B and the distance between A' and B' are almost the same. However, in the case where the finger turns in the finger axis direction, as shown in FIG. 7B, the finger of the user is flatter so that the difference between the A-B distance and the A'-B' distance becomes large.

When the difference between the A-B distance and the A'-B' distance exceeds a threshold value, it is determined that the finger turns, a guidance is output to put the finger correctly so that the user can put the finger in the correct position, and data is obtained again. By comparing the position data of the touch sensor 5 with contour data extracted from an image captured by the infrared camera 6, turn around the finger axis direction as a center can be detected.

As described above, by mounting the touch sensor on the fingertip side, the finger placement position, turn in the same plane using the optical axis of the camera as an axis, and turn around the finger axis direction as a center are detected. The vein data is corrected, and the guidance to put the finger properly is given to the user. Therefore, the high-precision and user-friendly finger vein authentication unit can be realized.

### (Second Embodiment)

In a small-sized open-type finger vein authentication unit, an image pickup window W is set narrower than the finger. By completely close the image pickup window W with the finger, the influence of outside light is eliminated. In this case, the image pickup window W is small and only the part of an image acquisition area can be imaged, so that the finger contour line cannot be obtained in the image of the infrared camera 6. In this case, by mounting a touch sensor also on the finger base side, a turn of the finger can be detected without contour data.

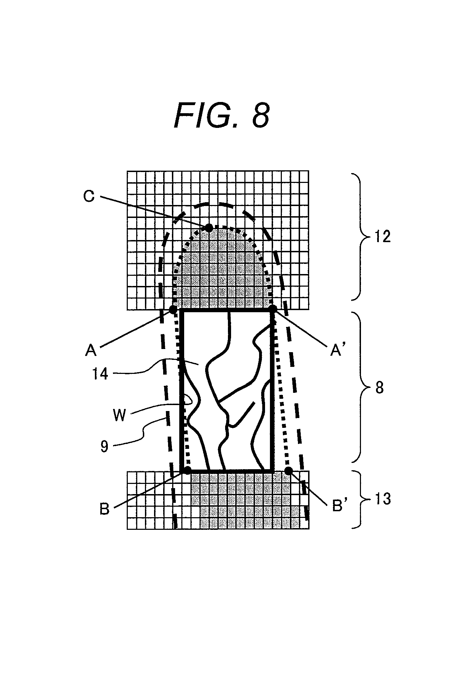

As shown in FIG. 8, the turn angle of the finger is calculated from a tilt of a line A-B connecting points A and B and a tilt of a line A'-B' connecting points A' and B' of a finger contact face detected by a fingertip touch sensor detection area 12 and a base touch sensor detection area 13 provided on the upper and lower sides of the infrared camera detection area 8. On the basis of the turn angle, an image captured in an image acquisition area 14 is turned. A deviation in the finger axis direction from a point C at the fingertip apex part of the touch sensor 5 on the fingertip side is also calculated, and the image is moved in the finger axis direction.

A portion in the finger to which the vein pattern in the image acquisition area 14 corresponds is determined from the data obtained as described above and, on the basis of the portion, matching with the collation template is performed. In such a manner, the high-precision finger vein authentication unit can be realized.

In the conventional technique, the portion in the finger to which the vein pattern obtained from the image pickup window cannot be known. Consequently, in the case where the finger is deviated or turns, authentication cannot be performed after matching the position of a vein pattern to be authenticated and the position of a vein pattern registered. There is the possibility that false acceptance such that a pattern of another person matches or false rejection such that the user is recognized as another person occurs, and the recognition rate deteriorates. However, when the portion in the finger to which the vein pattern corresponds can be determined as in the embodiment, authentication can be performed after matching the position of a vein pattern to be recognized with the position of a registered vein pattern. Therefore, deterioration in the authentication rate caused by deviation or turn of the finger can be suppressed.

### (Third Embodiment)

When the force of pressing the finger is strong, the fingertip becomes flatter, the contact face with the touch sensor 5 increases, and data detected by the touch sensor 5 becomes different from that at the time of registration. On the contrary, when the force of pressing the finger is weak, the contact face with the touch sensor 5 becomes smaller, and data detected by the touch sensor 5 may differ fromdata at the time of registration. When data detected by the touch sensor 5 at the time of registration and that at the time of authentication differs, it may deteriorate the authentication rate.

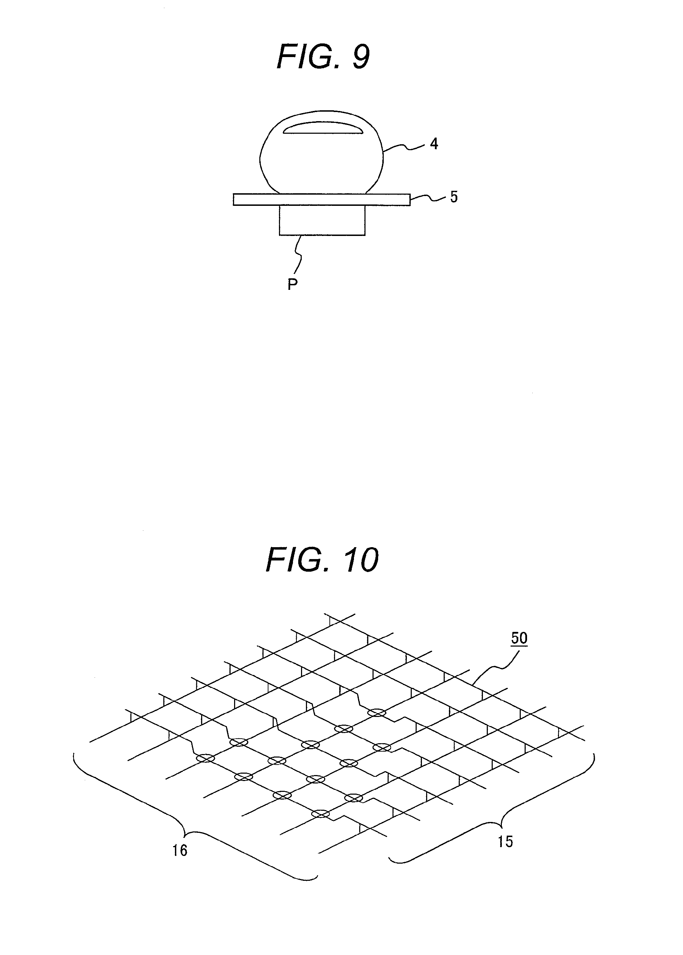

To avoid such a case, as shown in FIG. 9, a pressure sensor P is mounted just below the touch sensor 5, and a force of the finger 4 pressing the touch sensor 5 is detected. A range of the pressure which can be registered/authenticated is determined in advance. When the force is higher than the range, a guidance is output to put the finger lightly. On the contrary, when the force is lower than the range, a guidance is output to put the finger stronger. In such a manner, the way the finger is put at the time of registration can be reproduced more faithfully, and the authentication rate can be improved.

### (Fourth Embodiment)

Also when a contact-type touch sensor 50 in which registration films and electrodes are disposed in an X-Y matrix is used in place of the touch sensor 5 of the static-electric type used for detecting the position and shape of the fingertip in the finger vein authentication units of the first to third embodiments, the position and shape of the fingertip can be detected in a manner similar to the touch sensor of the static-electric type.

As shown in FIG. 10, the touch sensor 50 of the contact type has a structure that X electrodes 15 in the X direction and Y electrodes in the Y direction are overlapped in the vertical direction, and lower and upper electrodes only in a portion to which pressure is applied from above by a finger which is put come into contact with each other. By sequentially applying voltage to the X electrodes 15 and measuring the voltage of the Y electrodes 16 each time, the position and shape of the finger which is in contact can be detected.

Features, components and specific details of the structures of the above-described embodiments may be exchanged or combined to form further embodiments optimized for the respective application. As far as those modifications are readily apparent for an expert skilled in the art they shall be disclosed implicitly by the above description without specifying explicitly every possible combination, for the sake of conciseness of the present description.

## Claims

1. A finger vein authentication unit for performing biometric authentication by capturing a finger vein pattern (P) from an image pickup window (W) by an infrared camera (6) using near-infrared light,
wherein a touch sensor (5) having a plurality of detection regions which are continuous in XY directions is mounted in a fingertip placement part provided on the outside (7; 12) of the image pickup window (W), and
a finger displacement amount from a correct finger placement position in the finger vein authentication unit (101) is detected from fingertip shape data detected by the touch sensor (5) and finger contour data detected by the infrared camera (6), a finger vein pattern (P) obtained is corrected, and the same finger vein pattern (P) as that at the time of registration to the finger vein authentication unit (101) is automatically obtained.

2. The finger vein authentication unit according to claim 1, wherein the finger vein authentication unit (101) is configured to detect the finger displacement amount which corresponds to a deviation caused by turn of a finger in the same plane around an optical axis of the infrared camera (6) as a center.

3. The finger vein authentication unit according to claim 1 or 2, wherein the finger vein authentication unit (101) is configured to detect the finger displacement amount which corresponds to a positional deviation in a direction of turn around the axis of the finger as a center.

4. The finger vein authentication unit according to at least one of claims 1 to 3, wherein the finger vein authentication unit (101) is configured to detect the finger displacement amount which corresponds to a positional deviation in the axis directions of the finger.

5. The finger vein authentication unit according to at least one of claims 1 to 4, wherein the finger placement part has an almost flat shape.

6. The finger vein authentication unit according to at least one of claims 1 to 5, wherein when the finger displacement amount exceeds a predetermined threshold value, guidance information is output and notified to the user so that the user can put his/her finger in a correct placement position.

7. A finger vein authentication unit according to at least one of claims 1 to 6, wherein a second touch sensor having a plurality of detection regions which are continuous in XY directions is mounted in a finger base placement part provided on the outside (13) of the image pickup window, and
a finger displacement amount from a correct finger placement position is detected from fingertip shape data and finger base shape data detected by the first and second touch sensors, correction is made, and the same finger vein pattern (P) as that at the time of registration to the finger vein authentication unit is automatically obtained.

8. A finger vein authentication unit for performing biometric authentication by capturing a finger vein pattern (P) from an image pickup window (W) which is closed with a finger by an infrared camera (6) using near-infrared light,
wherein a first touch sensor having a plurality of detection regions which are continuous in XY directions is mounted in a fingertip placement part provided on the outside (12) of the image pickup window (W),
a second touch sensor having a plurality of detection regions which are continuous in XY directions is mounted in a finger base placement part provided on the outside (13) of the image pickup window, and
a finger displacement amount from a correct finger placement position is detected from fingertip shape data and finger base shape data detected by the first and second touch sensors, correction is made, and the same finger vein pattern (P) as that at the time of registration to the finger vein authentication unit is automatically obtained.

9. The finger vein authentication unit according to claim 7 or 8, wherein the finger displacement amount corresponds to a deviation caused by turn of a finger in the same plane around an optical axis of the infrared camera (6) as a center.

10. The finger vein authentication unit according to at least one of claims 7 to 9, wherein the finger placement part has a flat shape or an almost flat shape.

11. The finger vein authentication unit according to at least one of claims 7 to 10, wherein when the finger displacement amount exceeds a predetermined threshold value, guidance information is output and notified to the user so that the user can put his/her finger in a correct placement position.

12. The finger vein authentication unit according to at least one of claims 1 to 11, wherein the touch sensor (5) or at least one of the first and second touch sensors is provided with a pressure sensor for detecting a pressing force to the touch sensor.

13. The finger vein authentication unit according to at least one of claims 1 to 12, wherein the touch sensor 5) or at least one of the first and second touch sensors is a resistive touch sensor.
